# EUROPÄISCHE PATENTSCHRIFT

(11) **EP 2 243 022 B1**
(45) Veröffentlichungstag und Bekanntmachung des Hinweises auf die Patenterteilung: **04.01.2012**
(21) Anmeldenummer: 09709690.3
(22) Anmeldetag: 11.02.2009
(51) Int. Cl.: G01N 27/00, G01N 33/22

(54) **VORRICHTUNG UND VERFAHREN ZUM NACHWEIS VON TRIACETONTRIPEROXID**
APPARATUS AND METHOD FOR DETECTING TRIACETONE TRIPEROXIDE
DISPOSITIF ET PROCEDE DE DETECTION DE TRIPEROXYDE DE TRICYCLOACETONE

(30) Priorität: 11.02.2008 DE 102008008660
(43) Veröffentlichungstag der Anmeldung: 27.10.2010
(73) Patentinhaber: Rheinische Friedrich-Wilhelms-Universität Bonn, 53113 Bonn (DE); Max-Planck-Gesellschaft zur Förderung der Wissenschaften e.V., 80539 München (DE)
(72) Erfinder: WALDVOGEL, Siegfried, R., 53123 Bonn (DE); LÖRGEN, Jürgen, 68307 Mannheim (DE); LUBCZYK, Daniel, 53129 Bonn (DE); MÜLLEN, Klaus, 50939 Köln (DE); BAUER, Roland, 85386 Eching (DE)
(74) Vertreter: von Kreisler Selting Werner
(86) Internationale Anmeldenummer: PCT/EP2009/051594
(87) Internationale Veröffentlichungsnummer: WO 2009/101118

(56) Entgegenhaltungen:
- US-A1- 2006 188 399
- US-A1- 2006 191 320
- US-B1- 6 316 268
- US-B2- 7 159 463

## Beschreibung

Die vorliegende Erfindung betrifft eine Vorrichtung und ein Verfahren zum schnellen und sicheren Online-Nachweis von Triacetontriperoxid (TATP).

### Hintergrund der Erfindung

Triacetontriperoxid (TATP) ist ein sehr einfach herstellbarer Sprengstoff, dessen Ausgangsstoffe aus Drogerieartikeln leicht verfügbar sind (J. Zabicky, The chemistry of peroxides, John Wiley & Sons, Ltd; (2006); J.P. Agrawal, R.D. Hodgson, Organic Chemistry of Explosives, John Wiley & Sons, Ltd; (2007)). Zudem, lassen sich die Zutaten (Wasserstoffperoxid, Aceton und Säure) leicht in großer Menge beschaffen. Weil er sehr empfindlich auf Hitze, Stoß und Elektrostatik reagiert sowie keine Initialzündung benötigt und zudem eine vergleichbare Sprengkraft wie TNT besitzt, hat er keine kommerzielle oder militärische Bedeutung. Er wird allerdings aufgrund der leichten Zugänglichkeit häufig zur terroristischen Zwecken (z.B. Madrid März 2004, London Juli 2005) eingesetzt. Aus diesem Grunde ist ein einfacher und schneller Online-Nachweis zur Gefahrenabwehr notwendig. Zurzeit sind folgende Nachweismethoden bekannt (J.W. Gardner, J. Yinon (Eds.), Electronic Noses & Sensors for the Detection of Explosives, Kluwer Academic Publishers (2003); US 7,159,463; Z. Lin et al., Anal. Chem. 65, 1546-1551 (1993); E. Uttenthaler et al., Biosensors & Bioelectronics 16, 735-743 (2001); DE-A-10109534):
Trennung über RP-HPLC: Auftrennung einer Probenlösung über eine RP-HPLC, Zersetzung des TATP zu Wasserstoffperoxid mit einer UV-Lampe und Bestimmung der Wasserstoffperoxid-Konzentration mittels Fluoreszenzspektroskopie. Mit dieser Methode ist keine Onlinemessung möglich, da erst eine Probenlösung erstellt werden muss, welche nur mit großem analytischem Aufwand weiter untersucht werden kann.

Photometrisch: Behandlung einer Probenlösung mit Peroxidase, um vorhandene Spuren an Wasserstoffperoxid zu zerstören, nachfolgende Bestrahlung dieser Lösung mit einer UV-Lampe, zur Zersetzung von TATP zu Wasserstoffperoxid, nach dem Zusatz einer Reagenzlösung wird die Probe abschließend photometrisch mit einem UV/Vis-Spektrometer untersucht. Diese Methode ist portabel, aber die Erzeugung einer Probenlösung notwendig. Außerdem ist sie analytisch und apparativ aufwendig und nicht für Online-Untersuchungen geeignet.

E-3500: Bei dieser Methode wird die Probe durch manuelles Aufwischen gesammelt! (eventuell erste Explosionsgefahr) Nach Aufbringen auf einer Metallscheibe wird der Analyt thermisch zersetzt. Durch die entstehenden oxidativen Zerfallsprodukte wird mit Luminol eine Chemolumineszenz erzeugt, die gemessen werden kann. Durch den notwendigen manuellen Aufwand ist keine Onlinemessung möglich und die Anwendung zur Massenpersonenkontrolle fraglich. Dieses System verfügt über keine ausreichende chemische Selektivität, da keine Auftrennung der Analyten stattfindet. Dadurch wird jede Substanz, die brandfördernde Zerfallsprodukte beim Erhitzen freisetzt, als TATP gewertet.

z-Nose: Die z-Nose ist eine Miniaturvariante einer Gaschromatographie mit einem nachgeschalteten SAW-Sensor (Surface accustic wave) als massensensitiven Detektor. Die Erkennung der Substanzen erfolgt über einen Vergleich der Retentionszeiten mit einem Alkanstandard. Auch bei dieser Methode erfolgt eine Voranreicherung der Probe vor der eigentlichen Analyse, wodurch keine Onlinemessung mehr möglich ist.

Mini-Nose: Die Mini-Nose ist ein System, welches sich aus zwei Modulen zusammensetzt. Das erste dieser Module wird zum sammeln und anreichern der Probe genutzt. Das zweite Modul analysiert die gesammelte Probe. Im zweiten Modul ist ein Sensorarray bestehend aus mehreren beschichteten HFF-Schwingquarzen enthalten. Zur Messung wird der zunächst gesammelte Analyt aus Modul eins heraus in Modul zwei desorbiert. Nach weiteren 60 s Messzeit wird die Substanz dann mittels Hauptkomponentenanalyse identifiziert. Auch in diesem Fall ist klar ersichtlich, dass durch den notwendigen Anreicherungsschritt keine Onlineuntersuchung möglich ist.

WO02/103340 beschreibt den Einsatz eines Schwingquarzarrays mit hochfrequenten Quarzen sog. HFF-Quarzen. Das bei jenem Verfahren verwandte Sensorarray ist allerdings so unempfindlich, dass vor dem eigentlichen Nachweis ein Anreicherungsschritt notwendig ist.

Jedes einzelne dieser Verfahren beinhaltet jedoch Nachteile, die es für eine Online-Überwachung untauglich machen: Es ist entweder analytisch aparativ aufwendig, besitzt eine mangelnde Nachweisgrenze (evt. Anreicherung notwendig), das Messintervall ist unzureichend lang, ist unselektiv vor chemisch ähnlichem Hintergrund oder es ist erforderlich, dass TATP in flüssiger Phase vorliegt. Dies bedeutet, dass bislang ein Nachweis von TATP-Spuren in der Luft nur über Anreicherung oder mit hohem analytischem Aufwand möglich ist. Dies verhindert eine Online-Überwachung von Personen und / oder Objekten zur Gefahrenabwehr. Ein einfacher und schneller Nachweis des Sprengstoffes TATP wäre somit sehr willkommen.

### Kurzbeschreibung der Erfindung

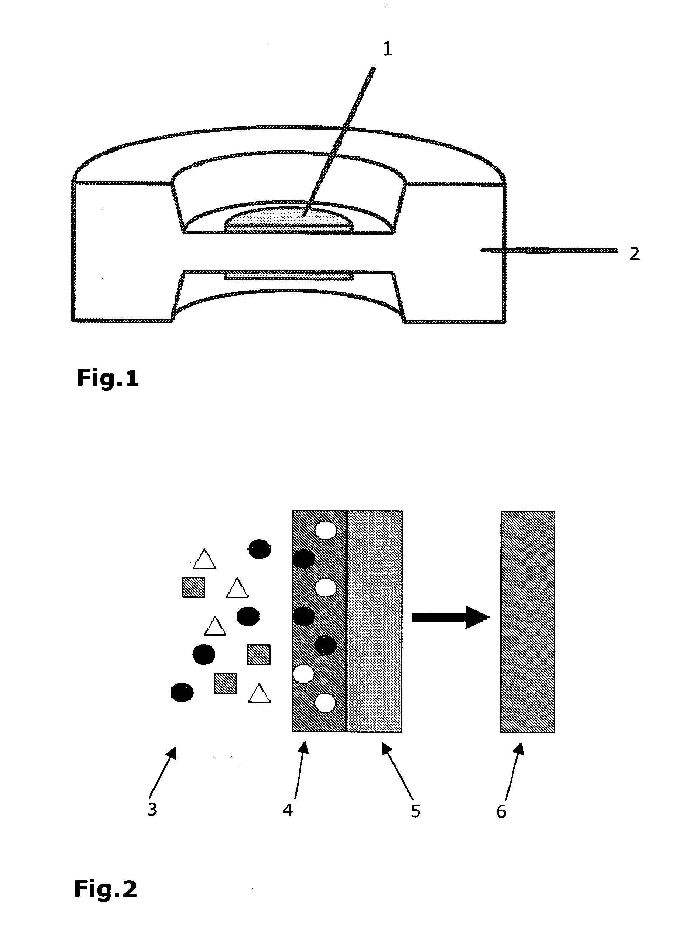

Es wurde nunmehr gefunden, dass ein Nachweis von TATP mittels einer Sensortechnologie auf Grundlage von HFF-Schwingquarzen erfolgen kann. Dieses sind im Prinzip Grundton-Schwingquarze, deren Materialstärke mittels der "Inverted mesa Technology" im Zentrum reduziert wurde (Figur 1). Der dünnere Bereich wird beidseitig mit metallischen Elektroden kontaktiert und unter Ausnutzung des umgekehrten piezoelektrischen Effekts mit einer Hochfrequenz, welche der Eigenfrequenz des Quarzes entspricht, angeregt. Die auf diese Weise erzeugte Resonanzschwingung ist direkt von der Masse des Oszillators abhängig. Masseänderungen, zum Beispiel durch Adsorption, führen daher zu Frequenzänderungen, welche leicht messbar sind. Die vorliegende Erfindung betrifft somit
(1) eine Vorrichtung zum Online-Nachweis von Triacetontriperoxid (TATP) umfassend eine Messzelle mit wenigstens drei unterschiedlich beschichtete HFF-Schwingquarzsensoren (Sensoren), wobei jeweils eine der sensitiven Flächen der drei Schwingquarze mit einem Cyclodextrinderivat, einem formstabilen Dendrimer und einem Cholsäurederivat als Detektorsubstanz beschichtet sind; und
(2) ein Verfahren zum Online-Nachweis von Triacetontriperoxid (TATP) umfassend In-Kontakt-Bringen einer Vorrichtung wie vorstehend unter (1) definiert mit dem zu überprüfenden Medium.

Die Vorrichtung und das Verfahren der Erfindung ermöglicht einen schnellen (d.h. direkten, "Online") und kontinuierlichen Nachweis auf des Explosivstoffes TATP aus der Raumluft. Hierdurch werden schnelle vor Ort Untersuchungen an Personen und Objekten auf TATP - Spuren möglich.

### Kurzbeschreibung der Figuren

Figur 1: Schematischer Aufbau eines HFF-Schwingquarzes mit Elektrode (1) und Quarz (2).
Figur 2: Prinzipieller Sensoraufbau gemäß dem Schlüssel-Schloss-Prinzip, wobei der Analyt (3) an einer selektiven Rezeptorschicht (4) bindet und über Transducer (5) ein Signal in der Elektrode (6) erzeugt.
Figur 3: Hauptkomponentenanalyse der fünf Analyten mit dem Sensorarray (#= Wasser, ∼= Wasserstoffperoxid, -= Bis-tert-butylperoxid, *= Aceton, += TATP).
Figur 4: Übersicht über die Verhältnisse der Sensorsignale von TATP und verscheidenen Querempfindlichkeiten (Analytenkonzentration 1 Vol.ppm).
Figur 5: Schematischer Aufbau der Beschichtungseinheit (P. Mashayekhi, Dissertation, Bonn, (2005)).
Figur 6: Schematische Darstellung einer Coulumbexplosion nach Doyle mit Hamilton-Spritze 7, Cone-jet 8 Satelliten Tropfen 9 und Mikro-Tropfen 10.
Figur 7: Schematischer Aufbau der Gasmischanlage.
Figur 8: Schematischer Aufbau der Messzelle.
Figur 9: Konzentrationsreihe eines mit 10 ng Octakis(2,3,6-tri-*O*-methyl)-γ-cyclodextrin beschichteten Quarzes mit TATP.
Figur 10: Anblasuntersuchungen bei verschiedenen Entfernungen (Beschichtung, Polyurethan 105 ng) mit einem Modell Analyt.
Figur 11: Zeitliche Erkennung eines TATP Signals bei einer Konzentration von 35 ppm mit dem optimalen Sensorarray.
Figur 12: Elektronenmikroskopische Untersuchung beschichteter Quarze. Beschichtung bei Normaldruck (links) und im reduzierten Umgebungsdruck (rechts)

### Detaillierte Beschreibung der Erfindung

In der Vorrichtung gemäß Aspekt (1) der Erfindung werden die Sensoren zur Erreichung einer chemischen Selektivität mit affinen Akzeptorsubstanzen, in definierten Schichtdicken, beschichtet. Dadurch erfolgt gemäß dem Schlüssel-Schloss-Prinzip eine Vorselektion der Analytmoleküle (Figur 2). Üblicherweise sprechen so geartete Sensoren nicht nur auf eine einzige Substanz, sondern auch auf chemisch ähnliche Substanzen an. Zur Eliminierung dieser Querempfindlichkeiten und zum sicheren Nachweis einzelner Substanzen werden mehrere Sensoren in einem Sensorarray kombiniert. Entsprechende Systeme sind in der Literatur bereits als "chemische Nasen" beschrieben.

Aufgrund dieser Erfahrungen werden mehrere Sensoren, welche verschiedenartig beschichtet sind, genutzt und deren Signale mit chemometrischen Methoden ausgewertet. Je unspezifischer die Sensorantworten sind, desto mehr Sensoren werden nötig. Die meisten in der Literatur beschrieben Arrays müssen sich allerdings zusätzlich verschiedener Anreicherungsverfahren bedienen, um die nötige Nachweisgrenze zu erreichen.

Die Erfindung bedient sich einer ähnlichen Technologie wie die beschriebene, allerdings entfällt - bedingt durch die spezielle Sensorkombination - eine zeitaufwändige Anreicherung. In Kombination mit leistungsfähigen Auswertroutinen ist ein schneller Nachweis in einem einzigen Messschritt ohne zusätzliche Anreicherung möglich.

Die vorliegende Erfindung beruht auf der Entwicklung einer Kombination von Beschichtungen, die hoch selektiv und reversibel auf TATP-Spuren ein Messsignal ergeben. Diese Selektivität sowie Sensitivität vereinfacht die Detektion von TATP gravierend.

Als Affinitätsmaterialien (Detektorsubstanzen) kommen nur Substanzen in Betracht, die lipophile Hohlraumstrukturen erzeugen (Arrays sind dann möglich wenn orthogonale Querempfindlichkeiten vorhanden sind). Die sichere Identifikation von TATP ist nur mit einer Kombination von mehreren unterschiedlich beschichteten Sensoren zu einem Sensorarray möglich. Die minimale Anzahl der benötigten Sensoren beträgt bei den hier mit untersuchten Querempfindlichkeiten drei.

Die Kombination an Affinitätsmaterialien, die bei den Sensoren der vorliegenden Erfindung eingesetzt werden, besteht aus einem Cyclodextrinderivat, einem formstabilen Dendrimer und einem Cholsäurederivat. Die auf die sensitive Fläche des Quarzes aufgetragenen Menge an Affinitätsmaterial ist dabei abhängig von der Art des Affinitätsmaterials, vorzugsweise sind die sensitiven Flächen der Quarze mit 10 bis 350 ng; vorzugsweise 25 bis 150 ng Detektorsubstanz beschichtet, was einer Frequenzerniedrigung von 100 bis 300 kHz entspricht.

Es ist dabei bevorzugt, dass das Cyclodextrinderivat ein β- oder γ-Cyclodextrinderivat, vorzugsweise Perallyl-γ-cyclodextrin ist.

Weiterhin ist bevorzugt, dass das formstabile Dendrimer ein Phenylen-Dendrimer, vorzugsweise ein pyridinhaltiges Terphenylen-Dendrimer der 4. Generation (Shifrina et al., Macromolecules 38(24) :9920-9932 (2005)) ist. Schließlich ist bevorzugt, dass das Cholsäurederivat ein Cholsäuresalz, vorzugsweise Natriumcholat ist.

Als leistungsstärkste Kombination hat sich eine Zusammenstellung von Natriumcholat (vorzugsweise mit einer Beschichtung des Quarzes von etwa 70 ng), Octakis(2,3,6-tri-*O*-allyl)-y-cyclodextrin (vorzugsweise mit einer Beschichtung des Quarzes von etwa 35 ng) und das pyridinhaltige Terphenylendendrimer MPI-7, d.h. das in Shifrina et al., Macromolecules 38(24):9920-9932 (2005)) beschriebene Dendrimer der 4. Generation mit der nachfolgend gezeigten Formel (Td-Gr-Ph) 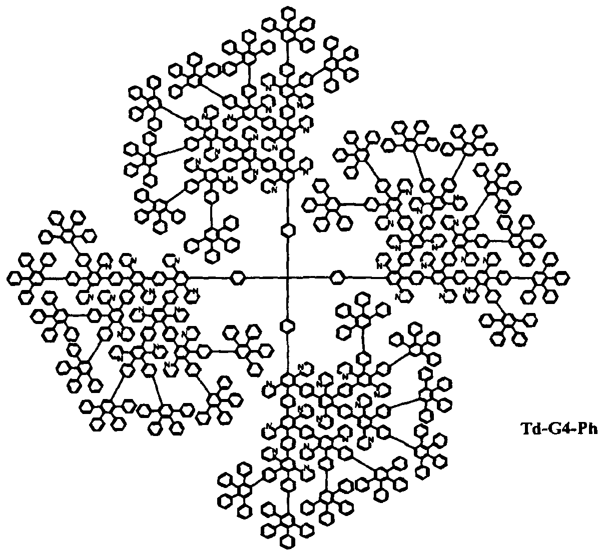 (vorzugsweise mit einer Beschichtung des Quarzes von etwa 105 ng) erwiesen, die im Folgenden und den experimentellen Beispielen als optimales Sensorarray bezeichnet wird. Einer der großen Vorteile des hier entwickelten Systems ist es, dass es dynamisch auf bis zu sechs Sensoren erweitert werden kann. Hierdurch ist es möglich das System ohne Eingriffe in die bestehende Arbeitsfähigkeit auch auf unerwartet auftretende Querempfindlichkeiten anzupassen.

Weitere Schwingquarzsensoren können z.B. mit nicht-quervernetztem Polyurethanen (z. B. solche die aus konventionellen Diisocyanaten wie Toluol-2,4-diisocyanat und Polyethylenglycol oder Polypropylenglycol hergestellt sind), Triphenylenketale (z.B. Triketale von Hexahydroxytriphenylen (Waldvogel et al., Angew. Chem. 112(14), 2580-83 (2000)) und/oder Porphyrinen (z. B. Metalloporphyrine wie Zn-Porphyrine) beschichtet sein.

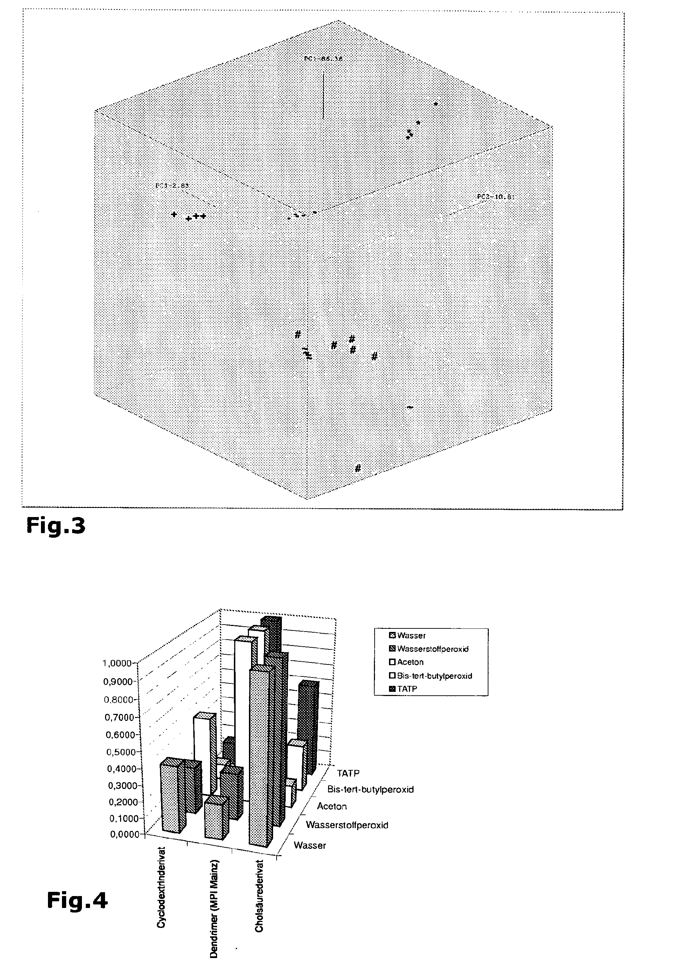

Das Sensorarray der vorliegenden Erfindung ist sehr selektiv und sehr empfindlich. Die Kombination mit der HFF-Schwingquarztechnologie und leistungsfähigen Auswertalgorithmen (Figur 3) erlaubt eine schnelle Online-Messung. Dies ermöglicht sehr einfach die Unterscheidung zwischen TATP und sowohl chemisch ähnlichen (Bis-*tert*-butylperoxid) als auch strukturell unähnlichen Querempfindlichkeiten (Figur 4). Die Erfindung ist damit schnell, kontinuierlich und selektiv.

Spezifische Ausführungsformen sind beispielsweise dadurch gekennzeichnet, dass die Signale der einzelnen Sensoren zur Minderung der Querempfindlichkeit (z.B. Aceton oder andere Peroxide) herangezogen werden. Der Auswertealgorithmus sorgt anschließend für ein selektives Messsignal zur Quantifizierung von TATP.

Zusätzlich sind die Arrays (Messeinheiten) sehr klein, so dass diese in bestehende Systeme integriert werden können. Auch eine Massenproduktion ist denkbar, da der geltende Materialpreis inkl. Elektronik wahrscheinlich unter € 500,- liegen wird.

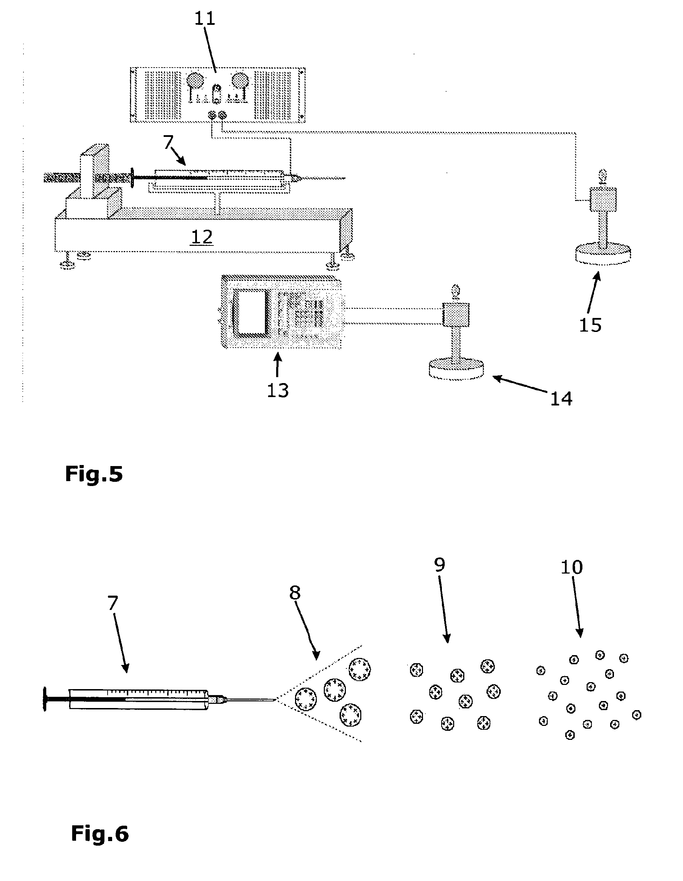

Die Beschichtung der sensitiven Flächen der Quarze erfolgte mit einem modifizierten Elektrosprayverfahren (DE-A-103 44 135). In Fig. 5 ist der schematische Aufbau einer für solch ein Verfahren geeigneten Beschichtungseinheit für Schwingquarze (QCM) gezeigt. Der Ablauf des Verfahrens erfolgt folgendermaßen: Die aufzutragende Substanz wird zunächst in einem flüchtigen, polaren Lösungsmittel, meist Methanol, gelöst, die Lösung in eine Hamilton-Spritze (7) aufgenommen und dann auf einem Schrittmotor (12) fixiert. Typischerweise werden Konzentration von 30 µg/l in einer 100 µl Spritze verwendet. An der Spritzenkanüle wird der Pluspol angelegt und an der Elektrode des Zielquarzes (15) der Minuspol. Zwischen diesen wird via Potentiometer (11) eine Hochspannung von etwa 5 - 6 kV angelegt. Der Schrittmotor drückt die Lösung langsam aus der Spritze. Dabei wird die Lösung durch die angelegte Spannung positiv aufgeladen und zur Elektrode des Quarzes (14) beschleunigt. Gemäß der in Coulombexplosion nach Doyle (DE-A-103 44 135; J. B. Fenn, Angew. Chem. 115, 3999 - 4024 (2003), s. Fig. 6) werden auf der Strecke zwischen Nadel und Elektrode die Tröpfchen durch den Verlust von Lösungsmittelmolekülen verkleinert, wodurch die Ladungsdichte im Tropfen kontinuierlich erhöht wird und Satelliten-Tropfen entstehen. Dies hat zur Folge, dass ab einem kritischen Radius die Coulomb-Abstoßung die Oberflächenspannung überwiegt und die Tröpfchen regelrecht explodieren. Es bilden sich dadurch Mikrotröpfchen. Auf diese Weise gelangt nur noch die reine Substanz auf die Elektrode. Bei dieser Art der Beschichtung sind die Schichten selbstheilend, da durch die als erstes aufgetroffenen Rezeptormoleküle eine isolierende Schicht auf der negativ geladenen Elektrode gebildet wird.

Bei den verwendeten HFF-Quarzen kommt ein weiteres geringfügig geändertes Verfahren zum tragen. Der Spraykegel wird wie oben beschrieben erzeugt, wobei der eigentlich zu beschichtende Quarz 10 - 15 cm neben dem Zentrum des Spraykegels platziert wird. Dies ist immer dann nötig, wenn die große Massenempfindlichkeit der HFF-Quarze beim direkten Beschichten zum sofortigen Zusammenbruch der Schwingung führen würde. Durch die geeignete Wahl eines der beiden Verfahren ist es möglich auf den Quarzen Beschichtungen zwischen 0 und 1000 kHz zu erzeugen. Mit diesem Verfahren können auf den Quarzen Beschichtungen zwischen 0 und etwa 350 ng erzeugen werden. Die Beschichtungsstärke kann kontinuierlich mit einem Frequenzzähler (13) überwacht werden. Dies ermöglicht hoch reproduzierbare und vergleichbare Beschichtungen.

In einer bevorzugten Ausführungsform des Beschichtungsverfahren erfolgt die Elektrospraybeschichtung bei reduziertem Druck (bei etwa 200 mbar) wodurch die Tropfenbildung reduziert wird.

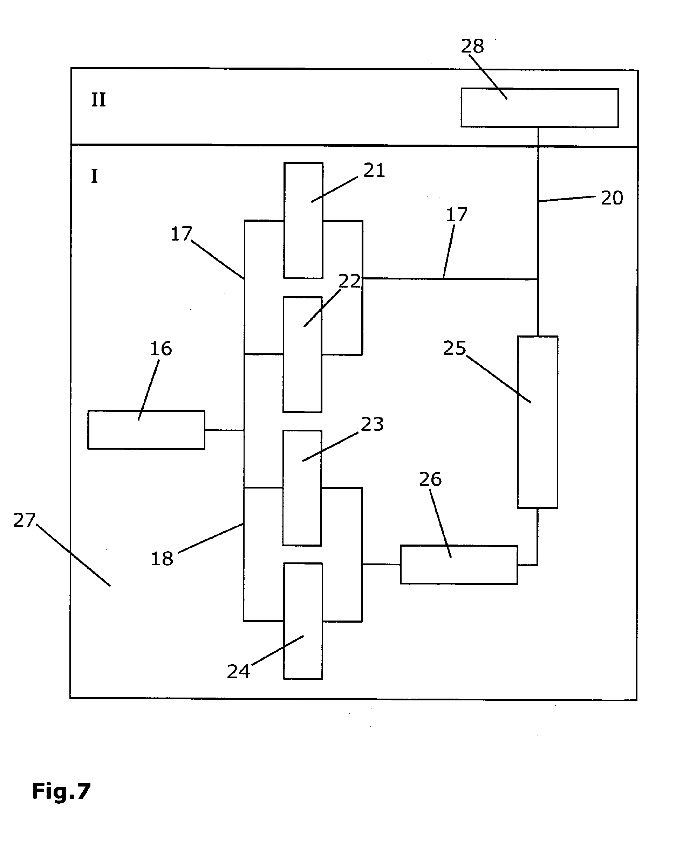

Zum Vergleich von unterschiedlichen Affinitäten sowie der Kalibrierung zwischen Analyt und Wirtssubstanz sind reproduzierbare Bedingungen essentiell. Daher wurde eine Gasmischanlage (siehe Figur 7) aus zwei Baugruppen (I, II) konstruiert, die diese Reproduzierbarkeit gewährleistet. Der erste Teil der Anlage (I) wird genutzt, um streng definierte Analytgasströme (20) zu erzeugen und bei Teil II handelt es sich um eine temperierte Messzelle (28) für sechs Quarzmikrowaagen, die an den Analytgasstrom (20) angeschlossen ist. Die Baugruppe I ist aus vier Mass-Flow-Controllern 5850S (21 = 200 ml/min, 22 = 20 ml/min) der Firma Brooks Instrument B.V. und einer auswechselbaren, auf 20 °C temperierten Gaswaschflasche aufgebaut. Mit den Mass-Flow-Controllern (21, 22) können aus einer Stickstoffflasche (16) zwei steuerbare Stickstoffgasströme (siehe 18 und 17) mit einem Volumen zwischen 1 - 200 ml/min entnommen werden. Während Strom (17) in Temperatur und Zusammensetzung unverändert bleibt, wird Strom (18) auf 20 °C temperiert (26) und durch die Gaswaschflasche (25) geführt. Durch die Rekombination beider Gasströme lässt sich ein Analytgasstrom (17) mit einem großen Spektrum an Analytkonzentrationen erzeugen. Die genaue Zusammensetzung des Gasstroms ist mit Hilfe einer seriellen Schnittstelle über einen Personal-Computer steuerbar.

Für Messungen von Proben, die über einen hohen Dampfdruck (∼100 ppm) verfügen beziehungsweise in großen Mengen eingesetzt werden können, wird folgendes Verfahren eingesetzt. Die Probengaserzeugung (20), indem der Stickstoffstrom (18) durch die mit Analyt gefüllte Gaswaschflasche geführt wird und hinter diesen mit dem reinen Stickstoffstrom (17) gemischt wird. Die Gaswaschflasche (25) wird auf 20 °C temperiert, da für die meisten Substanzen nur bei dieser Temperatur der genaue Dampfdruck bekannt ist, wodurch Berechnungen der Konzentration ermöglicht werden. Durch die Regelbarkeit der beiden Stickstoffströme (17, 18) mittels der Mass-Flow-Controller (21, 22) können Konzentrationen mit einem Volumenanteil zwischen 0,5 % und 100 % der maximalen Analytsättigung des Gasstroms erzeugt werden. Aus dem Mischungsverhältnis kann sehr leicht die volumenbezogene Konzentration in ppm berechnet werden.

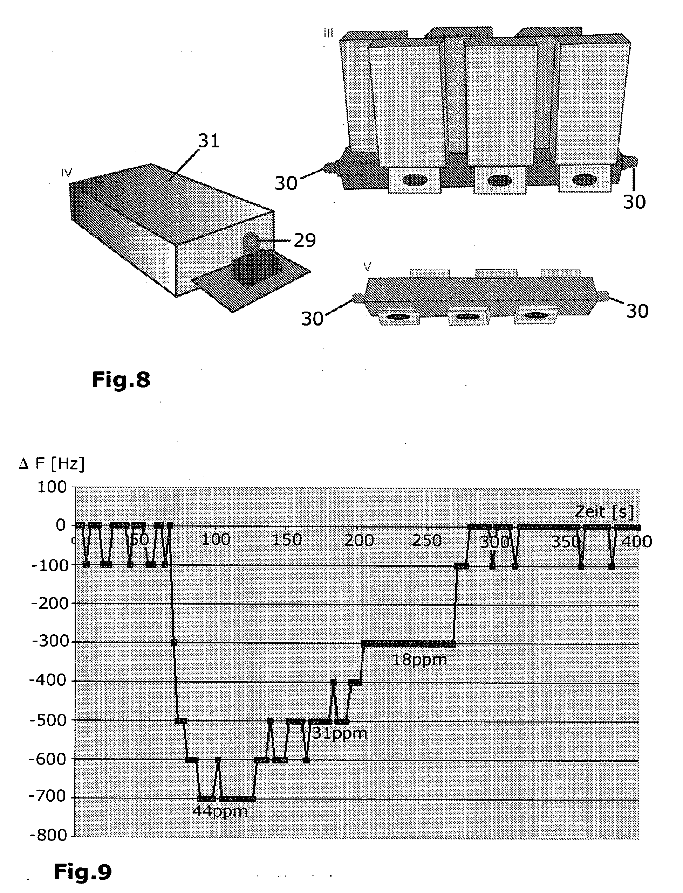

Die eigentliche Sensoreinheit (Fig. 8, III) setzt sich aus einem Stahlrohr (V) und sechs Sensormodulen (IV) zusammen. Die Sensormodule enthalten jeweils einen Oszillatorschaltkreis, mit dessen Hilfe der darauf montierbare Quarz (29) zum Schwingen anregt wird. Weiterhin bestehen sie aus über die USB-Schnittstelle digital auslesbaren Frequenzzählern. Diese Sensormodule werden in den Metallkorpus eingeführt und dicht verschraubt. Die so gebildete Messzelle besitzt an den Ausgängen Swagelok-Anschlüsse (30), über die sie mit Baugruppe I verbunden werden kann. Da die Quarzmikrowaagen (29) eine Temperaturabhängigkeit zeigen, wird die Messzelle konstant auf 35 °C gehalten. Durch die Wahl dieser Temperatur ist gewährleistet, dass sie immer oberhalb der Raumtemperatur ist. Diese Maßnahme verhindert Kondensationseffekte und gleichzeitig bleibt die Dynamik der Sorptionvorgänge vergleichbar.

Die verwendete Elektronik besteht aus einer Halterung für den Quarz (29), einem Oszillator (31), der den Quarz zum Schwingen anregt und einer Messeinheit (31), die mittels eines Prozessors die genaue Schwingungsfrequenz des Quarzes bestimmen kann (G. Sauerbrey, Verwendung von Schwingquarzen zur Wägung dünner Schichten und zur Mikrowägung, Z. Phys. 155, 206-222 (1959); Neubig, W. Briese, Das Grosse Quarzkochbuch, Franzis-Verlag Feldkirchen (1997)). Die Bauteile können über RJ 45 Anschlüsse in Reihe geschaltet werden und mittels eines USB-Adapters mit einem Personal-Computer ausgelesen werden.

In einer besonderen Ausführungsform der Erfindung ist vorgesehen, dass pro Quarz zwei Frequenzzähler vorgesehen sind. Es hat sich herausgestellt, dass bei heutzutage verfügbaren Frequenzzählern eine Torzeit von einer Sekunde für eine genaue Angabe ganzzahliger Frequenzen notwendig ist. Um eine höhere Abtastrate zu erzielen, werden daher die pro Quarz vorgesehenen Frequenzzähler im Abstand von 0,5 Sekunden gestartet und zeitversetzt ausgelesen. Selbstverständlich ist es möglich, auch mehr als zwei Frequenzzähler pro Quarz vorzusehen, die im unterschiedlichen Zeitabstand gestartet werden, um eine noch höhere Abtastrate zu erreichen.

Als Elektronik hat sich die Verwendung eines FPGA (field-programmable gate array) in 90 Nanometer-Technik als besonders vorteilhaft erwiesen, um die Zählergebnisse zu verarbeiten. Der Zählalgorithmus und die Weiterverarbeitung der Zählergebnisse werden innerhalb des FPGA durch die Kombination von einem VHDL-Design (Very High Speed Integrated Circuit Hardware Description Language) und eines IP-Softcores implementiert. Eine derartige Schaltung ermöglicht beispielsweise die parallele, asynchrone 28-bit Zählung von drei Sensoren mit einer Genauigkeit von ± ein bit. Dadurch ist eine Auflösung von bis zu einem Hertz möglich.

Die Vorrichtung zum Online-Nachweis von Triacetontriperoxid (TATP) kann beispielsweise ein Gehäuse mit innenliegendem Luftkanal aufweisen, wobei der Luftkanal mit einer Öffnung in dem Gehäuse verbunden ist. Durch diese Öffnung kann die mit Analyt behaftete Luft eingelassen werden. In dem Luftkanal können sich die Schwingquarze befinden, die mit der Elektronik verbunden sind.

Um eine verbesserte Ansaugung der Luft zu ermöglichen, kann eine Ansaugvorrichtung vorgesehen sein, die einen Unterdruck in dem Gehäuse erzeugt. Die Ansaugvorrichtung kann beispielsweise aus einem am Gehäuserücken angebrachten Ventilator oder durch eine an das Gehäuse angeschlossene externe Pumpe ermöglicht werden.

Da es notwendig ist, dass einströmende Luft ausschließlich über den Luftkanal den Schwingquarzen hinzugeführt wird, sollte das Gehäuse weitestgehend luftdicht ausgeführt sein.

Die erfindungsgemäße Vorrichtung kann auch als mobiles Gerät vorgesehen sein, die eine mobile Stromversorgung sowie eine drahtlose Datenübermittlung zur Übermittlung von Daten an einen Computer, beispielsweise einen WLAN-Adapter, aufweist.

Da Temperaturschwankungen zu Störungen im Signalverlauf führen können, kann vorgesehen sein, dass an der Öffnung des Luftkanals ein Aufsatz angeordnet ist, der einen Temperaturausgleich der einströmenden Luft ermöglicht. Dieser Aufsatz kann beispielsweise beheizbar sein, so dass die in den Luftkanal einströmende Luft durch Zuführung von externer Energie auf eine konstante Temperatur gebracht wird. Alternativ kann vorgesehen sein, dass in dem Aufsatz ein wärmeleitendes Material mit großer Oberfläche eingebracht ist, so dass die durch den Aufsatz einströmende Luft großflächig Kontakt mit dem Material bekommt, wodurch kleine Temperaturschwankungen durch intensiven Wärmeaustausch zwischen einzelnen Bereichen der einströmenden Luft erfolgreich reduziert werden können. Dafür kann beispielsweise vorgesehen sein, dass in dem Aufsatz mehrere Lochplatten aus Metall, beispielsweise Aluminium, angeordnet sind, durch die die einströmende Luft geleitet wird.

Die Erfindung wird anhand der nachfolgenden Beispiele näher erläutert. Diese schränken jedoch die Erfindung nicht ein.

### Beispiele

### Materialien und Methoden

Verwendete Quarze: HFF-Quarze im AT-Schnitt mit einer Grundfrequenz von 195 MHz auf einer HC-52/U Fassung. (Produkt Bezeichnung: Quarz * XA 1600 (ed. 06/05) RoHS-compliant Product, HC-52/U, QF: 195 MHz; Hersteller: KVG Quartz Crystal Technology GmbH, Neckarbischofsheim)

Beschichtungsverfahren: Die Beschichtung erfolgte mit einem modifizierten Elektrosprayverfahren (DE-A-103 44 135) mit der in Fig. 5 gezeigten Beschichtungseinheit für Schwingquarze (QCM). Die aufzutragende Substanz wurde zunächst in einem flüchtigen, polaren Lösungsmittel (Methanol) gelöst, die Lösung in eine Hamilton-Spritze mit einer Konzentration von 30 µg/l in einer 100 µl Spritze aufgenommen. An der Spritzenkanüle wurde der Pluspol angelegt und an der Elektrode des Zielquarzes der Minuspol. Zwischen diesen wurde via Potentiometer eine Hochspannung von etwa 5 - 6 kV angelegt, wobei der Schrittmotor die Lösung langsam aus der Spritze drückt. Dabei wurde die Lösung durch die angelegte Spannung positiv aufgeladen und zur Elektrode des Quarzes beschleunigt.

Der zu beschichtende Quarz wurde 10 - 15 cm neben dem Zentrum des Spraykegels platziert. Dies ist erforderlich, da die große Massenempfindlichkeit der HFF-Quarze beim direkten Beschichten zum sofortigen Zusammenbruch der Schwingung führen würde. Die Beschichtungsstärke wurde kontinuierlich mit einem Frequenzzähler überwacht.

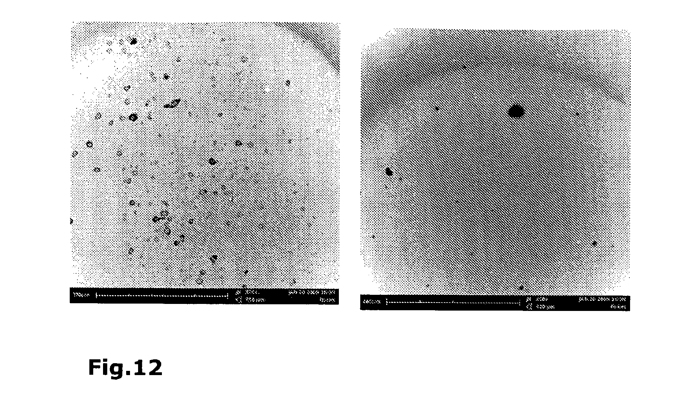

Das vorstehend beschriebene Beschichtungsverfahren birgt das Problem, das es kann zu erheblicher Tropfenbildung kommen kann, welche letztlich in einem inhomogenen Beschichtungsbild resultiert. Da dieses der Güte und Reproduzierbarkeit des Quarzes nicht zuträglich sein kann, wurde die Beschichtung bei reduziertem Druck durchgeführt. Hierzu wird die Spritzenkanüle in den Einlass eines evakuierbaren Glasgefäßes gebracht, der durch eine kontinuierlich laufende Pumpe auf einen Druck von 200 mbar gebracht wird. In diesem Glasgefäß befindet sich der zu beschichtende Quarz sowie die Gegenelektrode. Der weitere Aufbau bleibt wie dem vorstehend beschriebenen konventionellen Beschichtungsverfahren. In Fig. 12 wird ein durch dies modifizierte Verfahren beschichteter Quarz mit einem durch das konventionelle Verfahren hergestellten verglichen. Beschichtungen, welche mit dem modifizierten Verfahren hergestellt wurden, zeichnen sich durch eine deutlich geringere Zahl an großen Tropfenspuren auf der Oberfläche aus.

Aufbau der Messanlage: Zum Vergleich von unterschiedlichen Affinitäten sowie der Kalibrierung zwischen Analyt und Wirtssubstanz sind reproduzierbare Bedingungen essentiell. Daher wurde die in Figur 7 gezeigte, aus zwei Baugruppen (I, II) bestehende Anlage und die in Fig. 7, III Sensoreinheit verwendet.

Messverfahren: Das Prüfgas wurde mittels Verdünnung auf Konzentrationen zwischen 0,5 % und 100 % des maximal möglichen mit Analyt gesättigten Gasstroms eingestellt. Dadurch war es möglich die Isotherme bei 35°C zu bestimmen, deren Steigung in Hz/Vol.ppm mit den Isothermen anderer Analyten verglichen werden konnte.

Alle nachfolgenden ppm-Angaben beziehen sich auf diese Volumenverhältnisse. TATP-Probenerzeugung: Als TATP Quelle wurden "Echtstoff-Mikromengen-Prüfkörper (EMPK)" der Firma "Dülsner Sicherheitstechnik (Köln)" in zwei Ausführungen - 0,5 mg und 2,0 mg TATP - genutzt. Aufgrund dieser geringen Substanzmenge in Kombination mit der langsamen Desorptionsrate von den EMPK war es notwendig Gasproben einer definierten Konzentration zu erzeugen. Dazu wurden im Falle der mit 0,5 mg TATP beschichteten EMPK zwei Prüfkörper für mindestens 6 h in ein geschlossenes Glasgefäß (Lösungsmittelkugel aus der Säulenchromatographie) mit einem Volumen von 2,3 I gegeben. In dieser Zeit desorbiert das auf den Prüfkörpern befindliche TATP vollständig und es bildet sich eine Gasprobe mit einer definierten Konzentration von 44 ppm. Dieses Verfahren konnte genutzt werden, solange sich die Zielkonzentration unter dem Dampfdruck des TATPs von 68,5 ppm bei 25°C liegt. Bei den mit 2,0 mg TATP beschichteten EMPK wird nur ein Prüfkörper in ein Gefäß mit einem Volumen von 4,5 I gegeben. Durch die Desorption bildet sich ebenfalls ein Gasgemisch mit einer Konzentration von 44 ppm TATP. Diese Probengasbehälter können im Versuchsaufbau gegen die Gaswaschflasche ausgetauscht und durch Zumischen von Gasstrom (m) verdünnt werden.

Datenauswertung: Die Auswertung der Daten erfolgt in Echtzeit mittels Matlab (R2007b) der Firma "MathWorks" Hierbei werden die aufgenommenen Daten direkt normiert und damit nicht die absoluten Signale, sondern ihre Verhältnisse zueinander betrachtet werden können. Anschließend werden die so bearbeiteten Daten einer Hauptkomponentenanalyse (implementierte Funktion im Programm) unterzogen mit der die Signale einem Bereich in einem zweidimensionalen Koordinatensystem zugeordnet werden. Zur Substanzidentifikation des Analyten wird dieser so ermittelte Bereich mit denen definierter Analytproben verglichen. Affine Materialien: Es wird eine Komibination von Sensoren eingesetzt, die mit Natriumcholat (70 ng; kommerziell erhältlich von FLUKA), Octakis(2,3,6-tri-*O-*allyl)-γ-cyclodextrin (35 ng; A. Leydet et al., J. Med. Chem. 41, 4927-4932 (1998) und J. Ni et al., Carbohydr. Res. 337, 217-220 (2002)) bzw. Phenylendendrimer MPI-7 (105 ng; z. B. Shifrina et al., Macromolecules 38, 9920-9932 (2005)) beschichtet sind.

Messergebnisse: Für die Messung wurden kontinuierlich die Signale der einzelnen Sensorelemente aufgezeichnet und die Frequenzveränderung im Vergleich zur Grundlinie beobachtet. Für die Erstellung der Referenzdatensätze zur Analytidentifikation wurden mit den Rezeptorsubstanzen, in der beschriebenen Messanlage, Konzentrationsreihen für die Analyten gemessen und jeweils das Signal im Gleichgewicht bestimmt. Eine Konzentrationsreihe eines mit 10 ng Octakis(2,3,6-tri-*O*-allyl)-y-cyclodextrin beschichteten Quarzsensor TATP ist in Fig. 9 gezeigt.

### Beispiel 1: Affinitätsuntersuchung von Analyten

Es wurde die Affinität verschiedener Beschichtungen bezüglich TATP untersucht. Hierfür wurde neben Triacetontriperoxid (TATP) noch Wasser, Wasserstoffperoxidlösung (30%ig) und Aceton als potentielle Querempfindlichkeiten und außerdem Bis-tert-butylperoxid aufgrund seiner strukturellen Ähnlichkeit zum TATP betrachtet. Für diese fünf Analyten wurden Konzentrationsreihen bei einer Sensortemperatur von 35°C gemessen und jeweils die Isotherme bestimmt.

Der Konzentrationsbereich, in welchem die Analyten vermessen wurden, ergibt sich hauptsächlich aus seinem Dampfdruck bei 20°C. Eine Übersicht über die verwendeten Analyten, ihren Dampfdruck und die gemessenen Konzentrationsbereiche zeigt Tabelle 1.

**Tabelle 1: Übersicht über die untersuchten Analyten**

| Analyt | Dampfdruck [Vol. ppm] | Konz.-Bereich [Vol. ppm] |
|---|---|---|
| Wasser | 23000 (bei 20°C)* | 1150 - 6900 |
| H₂O₂ (30 %) | 14500 (bei 20°C)** | 725 - 4350 |
| Aceton | 245310(bei 20°C)*** | 12265 - 73593 |
| Bis-*tert*-butylperoxid | 53329 (bei 20°C)*** | 2666 - 15999 |
| Triacetontriperoxid | 68.5 (bei 25°C)**** | 3 - 44 |

| | | |
|---|---|---|
| *Herstellerangabe, Chemikalien Reagenzien, Merck (2003); **W. Schumb et al., Hydrogen Peroxide, Reinhold Publishing Corporation, New York (1955); ***Herstellerangabe, Handbuch Feinchemikalien, Aldrich (2007);**** J.C. Oxley, et al., Propellants, Explosives, Pyrotechnics 30, 2, 127-130 (2005). | | |

### Beispiel 2: Detektion im offenen System

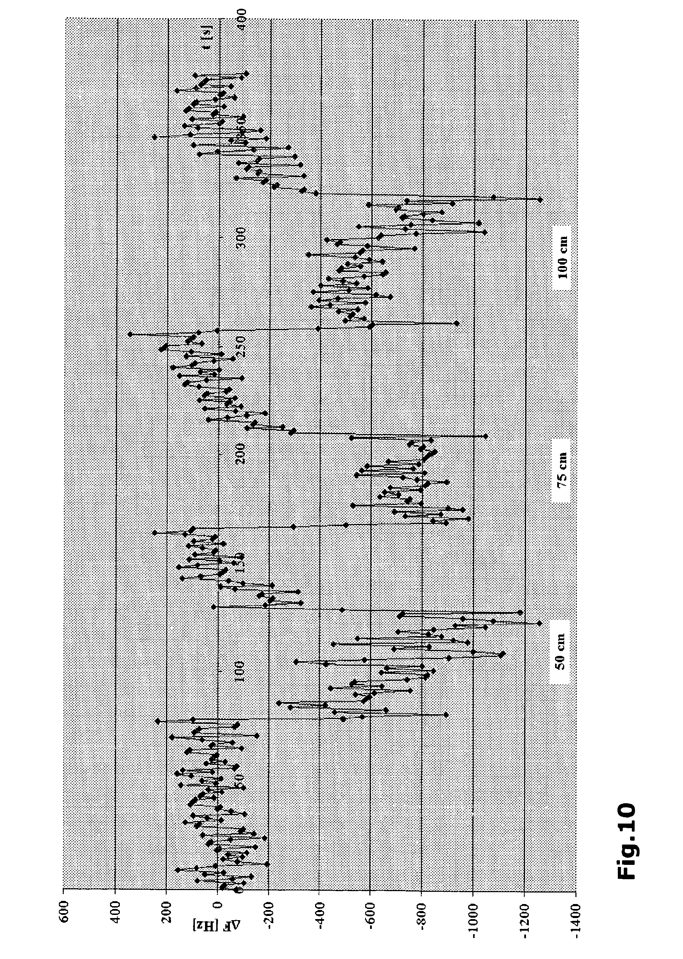

Für Messungen in einem offenen System mit einem Abstand zwischen Analytquelle und Sensorarray wurde ein offenes System konzipiert. Hierbei wird in einem Gang oder einer Schleuse gegenüber vom Sensor ein Gebläse installiert und ein auf den Sensor gerichteter Luftstrom erzeugt (ca. 2 m/s). Durch diesen Luftstrom werden Analytstoffe über eine Entfernung von ∼1 m sehr schnell und mit einer relativ kleinen Verdünnung zum Sensor befördert. Nachfolgend ist der Modellanalyt Phenol gezeigt, der einen vergleichbaren Dampfdruck wie TATP aufweist. Die Untersuchungen zeigen nahezu unabhängig von der Distanz ein Signalaufbau innerhalb von 2-4 Sekunden (Fig. 10).

### Beispiel 3: Zeitliche Auflösung Identifikation von TATP

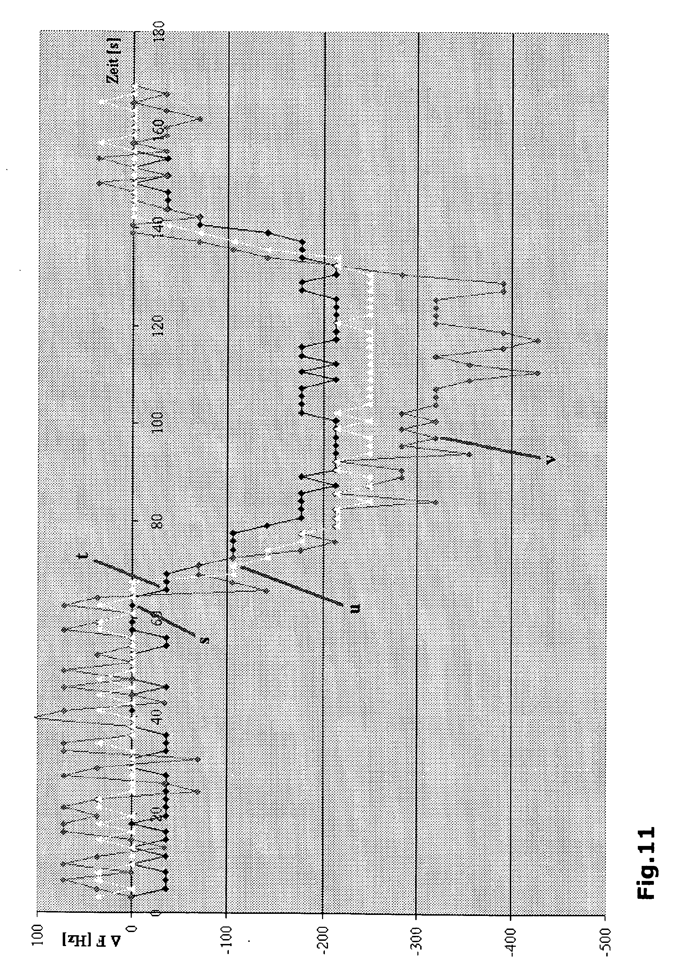

Das in diesem Patent beschriebene Sensorarray verfügt über sehr schnelle Ansprechzeiten zur sicheren Identifikation eines Signals. Drei Sekunden nach der Zugabe (s) ist bereits ein erstes Ansprechen (t) der Sensoren zu sehen. Bei diesen drei Sekunden handelt es sich um die apparativ bedingte Totzeit des Systems. Zum Zeitpunkt (u) (5 s später) sind die Signale bereits weit genug ausgeprägt für eine sichere Identifikation. Dieser Zeitpunkt liegt weit vor dem Gleichgewicht der Einlagerung, (v). Die Ergebnisse sind in Figur 11 zusammengefasst.

## Patentansprüche

1. Vorrichtung zum Online-Nachweis von Triacetontriperoxid (TATP) umfassend eine Messzelle mit wenigstens drei unterschiedlich beschichteten HFF-Schwingquarzsensoren (29), wobei jeweils eine der sensitiven Flächen der Schwingquarze mit einem Cyclodextrinderivat, einem formstabilen Dendrimer und einem Cholsäurederivat als Detektorsubstanz beschichtet sind.

2. Vorrichtung nach Anspruch 1, wobei
(i) das Cyclodextrinderivat ein β- oder γ-Cyclodextrinderivat, vorzugsweise Perallyl-γ-cyclodextrin ist; und/oder
(ii) das formstabile Dendrimer ein Phenylen-Dendrimer, vorzugsweise ein Terphenylen-Dendrimer der 4. Generation ist; und/oder
(iii) das Cholsäurederivat ein Cholsäuresalz, vorzugsweise Natriumcholat ist.

3. Vorrichtung nach Anspruch 1 oder 2, wobei die Messzelle einen oder mehrere weitere HFF- Schwingquarzsensoren enthält, die an den sensitiven Flächen mit nicht-quervernetzten Polyurethanen, Triphenylenketalen und/oder Porphyrinen beschichtet sind.

4. Vorrichtung nach einem oder mehreren der Ansprüche 1 bis 3, die einen mit Natriumcholat beschichteten HFF Schwingquarzsensor, vorzugsweise mit einer Beschichtung von etwa 70 ng, einen mit Octakis(2,3,6-tri-*O*-allyl)-γ-cyclodextrin beschichteten HFF Schwingquarzsensor, vorzugsweise mit einer Beschichtung von etwa 35 ng, und einen mit Phenylendendrimer (MPI-7) beschichteten HFF Schwingquarzsensor, vorzugsweise mit einer Beschichtung von etwa 105 ng, aufweist.

5. Vorrichtung nach einem oder mehreren der Ansprüche 1 bis 4, wobei die sensitiven Flächen der Quarze mit 10 bis 350 ng Detektorsubstanz beschichtet sind, was einer Frequenzerniedrigung der Quarze von 100 bis 300 kHz entspricht.

6. Vorrichtung nach Anspruch 5, wobei die Beschichtung durch ein Elektrosprayverfahren erfolgt, in dem das Auftragen der Substanz, gelöst in einem flüchtigen, polaren Lösungsmittel, durch Anlegen eine Hochspannung zur sensitiven Flächen des Quarzes beschleunigt wird.

7. Vorrichtung nach Anspruch 6, wobei das Elektrosprayverfahren unter reduziertem Druck erfolgt.

8. Vorrichtung nach einem oder mehreren der Ansprüche 1 bis 7, die weiterhin mindestens einen Frequenzzähler und mindestens eine Auswerteeinheit (31) enthält.

9. Vorrichtung nach Anspruch 8, wobei mindestens zwei Frequenzzähler pro Quarz vorgesehen sind, die vorzugsweise zeitlich versetzt betreibbar sind.

10. Verfahren zum Online-Nachweis von Triacetontriperoxid (TATP) umfassend In-Kontakt-Bringen einer Vorrichtung nach einem oder mehreren der Ansprüche 1 bis 9 mit dem zu überprüfenden Medium.

11. Verfahren nach Anspruch 10, das eine TATP-Empfindlichkeit von <10 ppm, vorzugsweise <3 ppm in der Raumluft aufweist, und keine Anreicherung des TATP erfordert.

12. Verfahren nach Anspruch 10 oder 11, das weiterhin den Abgleich mit einer Referenzsubstanz oder einem Referenzsubstanzgemisch umfasst.

## Claims

1. A device for the online detection of triacetone triperoxide (TATP), comprising a measuring cell with at least three differently coated HFF oscillating quartz crystal sensors (29), the sensitive surfaces of the three oscillating quartz crystals being coated with a cyclodextrin derivative, a dimensionally stable dendrimer and a cholic acid derivative, respectively, as detector substances.

2. The device according to claim 1, wherein
(i) said cyclodextrin derivative is a β- or γ-cyclodextrin derivative, preferably perallyl-y-cyclodextrin; and/or
(ii) said dimensionally stable dendrimer is a phenylene dendrimer, preferably a terphenylene dendrimer of the 4th generation; and/or
(iii) said cholic acid derivative is a cholic acid salt, preferably sodium cholate.

3. The device according to claim 1 or 2, wherein said measuring cell contains one or more further HFF oscillating quartz crystal sensors coated with non-cross-linked polyurethanes, triphenylene ketals and/or porphyrins on the sensitive surfaces.

4. The device according to one or more of claims 1 to 3, having an HFF oscillating quartz crystal sensor coated with sodium cholate, preferably with a coating rate of about 70 ng, an HFF oscillating quartz crystal sensor coated with octakis(2,3,6-tri-O-allyl)-γ-cyclodextrin, preferably with a rate of about 35 ng, and an HFF oscillating quartz crystal sensor coated with phenylene dendrimer (MPI-7), preferably with a coating rate of about 105 ng.

5. The device according to one or more of claims 1 to 4, wherein the sensitive surfaces of the quartz crystals are coated with 10 to 350 ng of detector substance, which corresponds to a frequency reduction of the quartz crystals of from 100 to 300 kHz.

6. The device according to claim 5, wherein said coating is effected by an electrospray method in which the application of the substance dissolved in a volatile polar solvent is accelerated by applying a high voltage towards the sensitive surfaces of the quartz crystal.

7. The device according to claim 6, wherein said electrospray method is effected under a reduced pressure.

8. The device according to one or more of claims 1 to 7, further containing at least one frequency counter and at least one evaluator unit (31).

9. The device according to claim 8, wherein at least two frequency counters per quartz crystal are provided, which preferably can be operated asynchronously.

10. A process for the online detection of triacetone triperoxide (TATP), comprising contacting a device according to one or more of claims 1 to 9, with the medium to be tested.

11. The process according to claim 10, which has a TATP sensitivity of < 10 ppm, preferably < 3 ppm, in the ambient air and requires no enrichment of the TATP.

12. The process according to claim 10 or 11, which further comprises a comparison with a reference substance or a mixture of reference substances.

## Revendications

1. Dispositif de détection en direct de triperoxyde de tricycloacétone (TATP) comprenant une cellule de mesure avec au moins trois capteurs à quartz oscillant HFF (29) présentant différents revêtements, dans lequel une des surfaces sensibles des quartz oscillants est respectivement revêtue d'un dérivé de cyclodextrine, d'un dendrimère de forme stable et d'un dérivé d'acide cholique en tant que substance détectrice.

2. Dispositif selon la revendication 1, dans lequel
i) le dérivé de cyclodextrine est un dérivé de β- ou γ-cyclodextrine, de préférence la perallyl-γ-cyclodextrine ; et/ou
ii) le dendrimère de forme stable est un dendrimère de phénylène, de préférence un dendrimère de terphénylène de 4ème génération ; et/ou
iii) le dérivé d'acide cholique est un sel d'acide cholique, de préférence le cholate de sodium.

3. Dispositif selon la revendication 1 ou 2, dans lequel la cellule de mesure contient un ou plusieurs autres capteurs à quartz oscillant HFF qui sont revêtus sur les surfaces sensibles de polyuréthanes, triphénylènecétals et/ou porphyrines qui n'ont pas subi de réticulation transversale.

4. Dispositif selon l'une ou plusieurs des revendications 1 à 3, qui comprend un capteur à quartz oscillant HFF revêtu de cholate de sodium, de préférence un revêtement d'environ 70 ng, un capteur à quartz oscillant HFF revêtu d'octakis(2,3,6-tri-*O*-allyl)-γ-cyclodextrine, de préférence un revêtement d'environ 35 ng, et un capteur à quartz oscillant HFF revêtu de dendrimère de phénylène (MPI-7), de préférence un revêtement d'environ 105 ng.

5. Dispositif selon l'une ou plusieurs des revendications 1 à 4, dans lequel les surfaces sensibles des quartz sont revêtues de 10 à 350 ng de substance détectrice, ce qui correspond à une perte de fréquence des quartz de 100 à 300 kHz.

6. Dispositif selon la revendication 5, dans lequel le revêtement est réalisé par un procédé d'électronébullisation, dans lequel le dépôt de la substance, dissoute dans un solvant volatil polaire, est accéléré par application d'une haute tension sur les surfaces sensibles du quartz.

7. Dispositif selon la revendication 6, dans lequel le procédé d'électronébullisation est réalisé sous pression réduite.

8. Dispositif selon l'une ou plusieurs des revendications 1 à 7, qui comprend en outre au moins un compteur de fréquences et au moins une unité d'évaluation (31).

9. Dispositif selon la revendication 8, dans lequel au moins deux compteurs de fréquences par quartz sont prévus, que l'on peut de préférence faire fonctionner de manière décalée dans le temps.

10. Dispositif de détection en direct de triperoxyde de tricycloacétone (TATP) comprenant la mise en contact d'un dispositif selon l'une ou plusieurs des revendications 1 à 9 avec le milieu à contrôler.

11. Procédé selon la revendication 10 qui présente une sensibilité au TATP inférieure à 10 ppm, de préférence inférieure à 3 ppm dans l'air ambiant et ne nécessite pas l'enrichissement du TATP.

12. Procédé selon la revendication 10 ou 11, qui comprend en outre l'équilibrage avec une substance de référence ou un mélange de substances de référence.
